# EUROPEAN PATENT SPECIFICATION

(11) **EP 1 523 503 B1**
(45) Date of publication and mention of the grant of the patent: **15.04.2009**
(21) Application number: 03765177.5
(22) Date of filing: 23.07.2003
(51) Int. Cl.: C07K 16/00, A61K 39/395

(54) **THERAPEUTIC ANTIBODIES WITH REDUCED SIDE EFFECTS**
THERAPEUTISCHE ANTIKÖPER MIT REDUZIERTEN NEBENWIRKUNGEN
ANTICORPS THERAPEUTIQUES A EFFETS SECONDAIRES REDUITS

(30) Priority: 23.07.2002 US 397934 P
(43) Date of publication of application: 20.04.2005
(73) Proprietor: ISIS INNOVATION LIMITED, Summertown, Oxford OX2 7SG (GB)
(72) Inventor: WALDMANN, Herman, Oxford, Oxfordshire OX2 7QY (GB); FREWIN, Mark, Raymond, Oxford OX4 2JJ (GB); GILLILAND, Lisa, Kim, Balerno EH14 7NN (GB); DA SILVA GRACA, Luis, Richardo, Simoes, Oxford OX2 6UD (GB)
(74) Representative: Lord, Hilton David
(86) International application number: PCT/GB2003/003156
(87) International publication number: WO 2004/009638

(56) References cited:
- WO-A-02/066058
- US-A- 5 990 286
- US-A1- 2002 048 578
- GILLILAND L K ET AL: "Elimination of the Immunogenicity of Therapeutic Antibodies" , JOURNAL OF IMMUNOLOGY, THE WILLIAMS AND WILKINS CO. BALTIMORE, US, VOL. 162, PAGE(S) 3663-3671 XP002212906 ISSN: 0022-1767 the whole document
- HALE G: "Synthetic peptide mimotope of the CAMPATH-1 (CD52) antigen, a small glycosylphosphatidylinositol-anchored glycoprotein" , IMMUNOTECHNOLOGY, ELSEVIER SCIENCE PUBLISHERS BV, NL, VOL. 1, NR. 3, PAGE(S) 175-187 XP004052720 ISSN: 1380-2933 the whole document
- ISAACS J D ET AL: "HELPLESSNESS AS A STRATEGY FOR AVOIDING ANTIGLOBULIN RESPONSES TO THERAPEUTIC MONOCLONAL ANTIBODIES" , THERAPEUTIC IMMUNOLOGY, BLACKWELL SCIENTIFIC PUBL. LONDON, GB, VOL. 1, NR. 6, PAGE(S) 303-312 XP000673232 ISSN: 0967-0149 the whole document

## Description

This application claims priority based on application US 2002 0 397 934, filed July 23, 2002.

### FIELD OF THE INVENTION

The present invention relates to therapeutic antibodies and to a method for reducing the side effects thereof; in particular, those that result from cytokine release.

### BACKGROUND

In protein therapy, such as antibody therapy, the use of the antibody results in unwanted side effects; for example, those that result from cytokine release. As a result, there is a need for therapeutic proteins with reduced side effects.

### STATEMENT OF THE INVENTION

Thus, in accordance with the invention, there is provided a pharmaceutical comprising:
(a) a therapeutic antibody that includes an antibody combining site that binds to a therapeutic target, said antibody being modified with a peptide that inhibits binding of the antibody to the therapeutic target, said modified antibody being effective for reducing side effects caused by the antibody and for producing a therapeutic effect by binding to the therapeutic target; and
(b) a pharmaceutical carrier;
wherein the peptide is linked by a linker to the antibody and is reversibly bound to the antibody combining site.

Reference hereinafter to the compound will be understood to refer to the peptide. The therapeutic antibody is modified to include a compound that is reversibly bound to the antibody combining site (ACS) of the antibody, with the target antigen competing with the compound for binding to the ACS upon administration of the antibody, or the binding of the antigen otherwise being inhibited by the compound, whereby binding of the antibody to the target is inhibited. In this manner, the amount of the modified antibody that becomes bound to the target antigen in the initial period after administration is less than would have become bound if the antibody was administered in its non-modified form. As the compound is displaced from the ACS as a result of competitive binding, or the inhibitory affect of the compound is otherwise reduced, the amount of antibody that becomes bound to the target antigen increases. By inhibiting the binding of the antibody, with the amount of antibody that is bound to the target increasing over time, the modified antibody is capable of reducing and/or essentially eliminating side effects such as those that result from cytokine release and is also capable of accomplishing the desired therapeutic effect.

In one embodiment, the modified antibody has an avidity for the target that is less than the avidity for the target of the unmodified antibody. The avidity is reduced in an amount that is effective for reducing and/or eliminating side effects against the therapeutic antibody while producing the desired therapeutic effect by binding to the therapeutic target.

The term "therapeutic" as used herein encompasses both treating an existing disease condition or disorder and preventing and/or reducing the severity of a disease, condition or disorder.

A therapeutic target is the antigen to which the antibody binds, which antigen may or may not be present on a tissue or cells. The compound that is combined with the therapeutic antibody for inhibiting binding to the target may inhibit such binding by binding to the ACS and/or by binding or blocking access to the ACS; e.g., by steric hindrance.

The compound is combined with the antibody by linking the compound to the antibody and by binding of the compound to the ACS.

The compound is linked or tethered to the antibody and also binds to the ACS. In one non-limiting embodiment, the compound is linked to only one of the chains of the antibody.

The therapeutic antibody may be used as a therapeutic in humans and may be a non-human antibody e.g. one raised in a rodent.

The compound functions to inhibit binding of the antibody to the target whereby immediately after administration there is a limited reduction of the amount of antibody that binds to the target as compared to the amount of antibody that would bind without the presence of the compound. The amount of antibody that becomes bound to the target increases over time whereby in effect there is a temporary blocking of the ACS that inhibits the amount of antibody that binds to the target.

The temporary blockade of the ACS (a blockade that initially reduces the amount of antibody that binds to the target, with such amount increasing with time) may be effected by the following, including ;
(i) Temporary occupancy with molecules such as the defined antigen or a domain thereof, low affinity antigenic peptides or mimotopes by preincubation *in vitro,* that might gradually dissociates *in vivo*, such that the antibody would gradually accumulate on cell-bound or other "target" antigen if the association and dissociation constants were favourable by comparison with the "obstructive" element; or
(ii) Temporary occupancy with molecules such as the defined antigen or a domain thereof, low affinity antigenic peptides or mimotopes which may be attached by flexible linkers. Once administered *in vivo* the antibody would gradually accumulate on cell-bound or other "target" antigen if the association and dissociation constants were favourable by comparison with the "obstructive" element; or
(iii) Chemical drugs which may bind non-covalently in the ACS and be able to dissociate *in vivo;* or
(iv) Other changes that might temporarily obstruct the ACS; for example, temporarily blocking access to the ACS without the compound binding to the ACS.

Such a modification would interfere with antibody accumulation on the target antigen for a limited period, which would be enough to ensure that the administered therapeutic antibody has reduced side effects while allowing for the antibody to achieve the desired therapeutic effect, i.e., accumulate on the target antigen in an amount to produce such effect. Removal of the modification may also occur by the host's own physiological and biochemical processes such as pH changes, enzymatic cleavage within the host, natural competition with host antigens bound to cells. For example a peptide mimotope could be linked to the antibody H or L chain by a linker which carries an enzyme-degradable motif, susceptible to cleavage by host enzymes *in vivo,* such as for example, leukocyte elastase.

US-A-5 990 286 describes an antibody which is conjugated to a chemical reagent at at least one of a plurality of free amino groups, thereby producing a modified antibody. Whilst there have been modifications of the antibody taught in US-A-5 990 286, these modifications are clearly not intended to occur at the ACS (antibody combining site) of the antibody. This publication states that "by conjugation with a reagent that modifies free amino groups", an increase in antigen binding specificity can be achieved. However, it is clear that should this modification occur in the ACS, then an increase in binding specificity would not be achieved. Thus, the disclosure of US-A-5 990 286 is directed to increasing the binding specificity of the antibody by chemical modification of the antibody away from the ACS, in contrast to the present invention which is restricted to modification of the ACS. US-A-2002/0048578 discloses a humanized antibody which is less immunogenic than the non-modified antibody.

Preferably, the native antigen, domains thereof, and peptide fragments or mimotopes are used to modify the ACS. The latter may be generated from peptide libraries either synthetically or biologically-derived. Non-covalently binding chemicals can be screened from natural or synthetic libraries or from other available products, for their ability to inhibit antibody binding to its antigen or a surrogate equivalent. The linkers which may be used are preferably flexible.

The present invention is also directed to antibodies as described above further comprising an Fc region designed to reduce interaction with the complement system and with specialised cell receptors for the Fc region of immunoglobulin (FcR receptors). This will be useful for many antibodies where cell lysis is not essential, such as blocking or agonist antibodies.

When covalently linking the compound to the antibody, in one embodiment, the compound is preferably linked to only one of the two arms of the antibody.

The term "antibody" as used herein includes all forms of antibodies such as recombinant antibodies, humanized antibodies, chimeric antibodies, single chain antibodies, monoclonal antibodies etc. The invention is also applicable to antibody fragments that are capable of binding to a therapeutic target.

The compound (which may be a peptide or other molecule that is capable of binding to the ACS of the antibody) is reversibly bound to the antibody binding or combining site of the antibody that is to be administered to a person. The compound occupies the binding site of the antibody for the antigen and thereby inhibits binding of the antibody to the antigen. Since the compound is reversibly bound to the antibody binding site and is selected to have a limited reduction in antibody binding, the antibody is capable of binding to the antigen against which the antibody is directed.

In one embodiment, the compound that is selected for binding to the antibody combining site of the antibody is one whereby the antibody avidity for the compound is less than the antibody avidity for the antigen. In this manner, when the antibody is initially administered, there will be reduced binding of the antibody to the antigen, as compared to the binding that would occur in the absence of the compound, with such binding increasing over time.

Applicant has found that reduction of side effects (such as those resulting in cytokine release) to a therapeutic antibody can be accomplished by administering an antibody that is capable of effectively binding to the antigen producing the desired therapeutic effect, provided that during the period that immediately follows administration of the antibody, the amount of the antibody that binds to the antigen is reduced, with such amount being increased from the reduced amount over time.

Thus, unlike the prior art, in accordance with the invention, an antibody that is capable of performing its therapeutic function also reduces side effects, such as cytokine release caused by the antibody by initially reducing the amount of the therapeutic antibody binding to the antigen followed by an increase in the amount of the therapeutic antibody binding.

The compound that is used for binding to the antibody combining site in a manner that initially reduces the amount of antibody binding to the antigen is a peptide. The peptide may be identical to or different from a corresponding peptide portion of the antigen to which the therapeutic antibody binds. The appropriate peptide for an antibody may be selected by testing a panel of peptides in an inhibition of binding assay with respect to the antibody and its antigen. These and other procedures should be known to those skilled in the art based on the teachings herein.

In one embodiment, the antibody combined with the compound has an avidity for the target antigen that is less than the avidity of the non-modified antibody for the target antigen. The relative avidity of the modified antibody and the unmodified antibody may be determined by an inhibition of binding assay using fifty percent binding inhibition as an end point. A modified antibody has a reduced avidity if there is an increase in the amount of modified antibody as compared to the amount of unmodified antibody required to produce a fifty percent inhibition of the binding of a labelled unmodified antibody to the target antigen.

The avidity of the modified antibody is reduced in an amount that is effective for reducing and/or essentially eliminating side effects, in particular those caused by cytokine release and the modified antibody has an avidity for the target that is effective for producing the desired therapeutic effect. In general, the avidity is reduced by at least 4-fold and in general by no more than 100-fold. It is to be understood that there may be a greater reduction in avidity. Thus, in accordance with an aspect of the invention, the antibody is modified to reduce side effects and, in the case where it is desired to only reduce side effects, the reduction in binding is limited to an amount to achieve such result; i.e., side effect reduction can be achieved without reducing or eliminating an antibody response against the modified antibody.

In one non-limiting embodiment, the compound may inhibit binding of the modified antibody by providing a modified antibody with a reduced affinity for the target antigen as compared to the unmodified antibody. In one non-limiting embodiment, the modified antibody may have an affinity for the antigen to which it is to be bound that is at least two or at least five-fold less than the affinity of the unmodified antibody. In accordance with an embodiment of the invention, side effects can be reduced by reducing the affinity in a limited amount, for example, by no more than 100 fold and without eliminating an antibody response against the modified antibody. It is to be understood, however, that the affinity may be reduced in greater amounts, although, however, such greater reduction is not required in order to reduce side effects caused by cytokine release.

The modified antibody is employed in an amount that is effective for both producing the desired therapeutic effect and for reducing side effects. In general, without limiting the present invention, the modified antibody is administered in an amount such that the quantity of the antibody administered during the 24-hour period that begins when the antibody is first administered is at least 50 mg and in general at least 100 mg and more generally at least 200 mg.

The therapeutic antibody may be employed in combination with a pharmaceutically acceptable carrier. The use of a suitable carrier is deemed to be within the skill of the art from the teachings herein.

The present invention therefore provides a pharmaceutical comprising:
(a) a therapeutic antibody that includes an antibody combining site that binds to a therapeutic target, said antibody being modified with a peptide that inhibits binding of the antibody to the therapeutic target, said modified antibody being effective for reducing side effects caused by the antibody and for producing a therapeutic effect by binding to the therapeutic target; and
(b) a pharmaceutical carrier;
wherein the peptide is linked by a linker to the antibody and is reversibly bound to the antibody combining site, whereby the amount of antibody that binds to the target increases as the peptide is displaced from the antibody combining site.

It is preferred that the avidity of the modified antibody combined with the peptide is at least 4-fold less than the avidity of the unmodified antibody and no more than 100-fold less. Preferably, the antibody is an aglycosylated antibody. Even more preferably, only one of the chains of the antibody has a peptide linked thereto that binds to the antibody combining site.

The peptide is reversibly bound to the antibody combining site, whereby the amount of antibody that binds to the target increases as the compound is displaced from the antibody combining site. It is preferred that the Fc portion of the antibody is aglycosylated and more preferably, that the binding of the antibody to the Fc receptor is essentially eliminated.

Preferably, the antibody is a non-human antibody. It is also preferred that the antibody is a chimeric antibody.

The following non-limiting Examples illustrate the invention.

The humanised anti-CD52 antibody, CAMPATH-1H, was used in the following experiments. Various constructs were made using the CAMPATH-1H antibody and the following methodology.

### Generation of reduced-binding variants of CAMPATH-1H:

The cloning of the V-regions of the humanised antibody CAMPATH-1H specific for the human CD52 antigen is performed as described in Gilliland et al. 1999 The Journal of Immunology 162:33663-3671. The methodology is based on that of Orlandi et al. 1989 PNAS 86:3833, using the polymerase chain reaction (PCR). The wild-type humanised CAMPATH-1 light chain was cloned into the vector pGEM 9 (Promega) and used as a PCR template for site-directed mutagenesis.

A flexible linker (Gly4Ser x 2) was added to the amino-terminal end of the light chain between the CAMPATH-1H leader sequence and CAMPATH-1H VL sequence using the oligonucleotide primers PUCSE2 and Link L-3' + Link-L-5' and PUCSE REV. The resulting fragments were PCR assembled using primers PUCSE2 + PUCSE REV to give full length Linker-CP-1H light chain which could be cloned into expression vector as Hind111/Hind 111 fragment.

The Linker-CP-1H light chain construct was then used as a PCR template to generate the CD52 Mimotope (QTSSPSAD) and the CD52 Mimotope Mutant 9 (QTSAAAVD) constructs. Primers PUCSE2 and CD52MIM-3' + CD52MIM-5' and PUCSE REV were used to give Mimotope-CP-1H light chain construct. Primers PUCSE2 and MIMMut9-3' + MIMMut9-5' and PUCSE REV were used to give Mimotope Mutant 9-CP-1H light chain construct.

Linker-Only-CP-1H, Mimotope-CP-1H, and MimMut9-CP-1H mutants were transferred to pBAN-2, a derivative of the pNH316 mammalian expression vector containing neomycin selection (Page et al. 1991 Biotechnology 9:64-68) and PEE 12 a mammalian expression vector containing the Glutamine Synthetase gene for selection (Bebbington et al. 1992 Biotechnology 10:169-175).

Subconfluent dhfr⁻ Chinese Hamster Ovary cells (Page et al. 1991 Biotechnology 9:64-68) or NSO mouse myeloma cells (ECACC cat no 8511503, Meth Enzymol 1981, 73B,3) were co-transfected with the light chain mutants and the CAMPATH-1H heavy chain construct human IgG1 constant region.

CAMPATH-1H heavy chain constructs were expressed in pRDN-1, a variant of the pLD9 mammalian expression vector with a dhfr selectable marker (Page et al. 1991 Biotechnology 9:64-68), and PEE 12. Transfection was carried out using LipofectAMINE PLUS reagent (Life Technologies) following the manufacturers recommendations.

Human IgG1 constant region was derived from the wild type Glm (1,17) gene described by Takahashi et al. 1982 Cell 29: 671-679.

Heavy and Light chain transfectants were selected in hypoxanthine-free IMDM containing 1mg/ml G418 + 5% (v/v) dialysed foetal calf serum. Resulting selected cells were screened for antibody production by ELISA and for antigen binding to human T cell clone HUT 78 (Gootenberg JE et al. 1981 J. Exp. Med. 154:1403-1418) and CD52 transgenic mice.

Cells producing antibody were cloned by limiting dilution, and then expanded into roller bottle cultures. The immunoglobulin from approximately 15 litres of tissue culture supernatant from each cell line was purified on protein A, dialysed against PBS and quantified.

### List of Primers used

| | |
|---|---|
| PUCSE-2 | 5'-CAC AGA TGC GTA AGG AGA AAA TAC-3' (SEQ ID NO. 1). |
| PUCSE REV | 5'-GCA GTG AGC GCA ACG CAA T-3 (SEQ ID NO.2).' |
| LINK-L3' | 5'-GCT TCC GCC TCC ACC GGA TCC GCC ACC TCC TTG GGA GTG GAC ACC TGT AGC TGT TGC TAC-3' (SEQ ID NO. 3). |
| LINK-L5' | 5'-GGA GGT GGC GGA TCC GGT GGA GGC GGA AGC GAC ATC CAG ATG ACC CAG AGC CCA AG-3' (SEQ ID NO. 4). |
| CD52MIM-3' | 5'-GTC TGC TGA TGG GCT GCT GGT TTG GGA GTG GAC ACC TGT AGC TGT TGC-3' (SEQ ID NO. 5). |
| CD52Mim-5' | 5'-CAA ACC AGC AGC CCA TCA GCA GAC GGA GGT GGC GGA TCC GGT GGA GGA-3' (SEQ ID NO. 6). |
| MimMut9-3' | 5'-GTC TAC TGC TGC GGC GCT GGT TTG GGA GTG GAC ACC TGT AGC TGT TGC-3' (SEQ ID NO. 7). |
| MimMut9-5' | 5'-CAA ACC AGC GCC GCA GCA GTA GAC GGA GGT GGC GGA TCC GGT GGA GGA-3' (SEQ ID NO. 8). |

### Constructs and Cell Lines produced

### TF CHO/CP-1H IgG1/MIM and TF NSO/CP-1H IgG1/MIM (MIM IgG1)

CD52 Mimotope QTSSPSAD tethered to CAMPATH-1H light chain V-region by flexible Glycine4 Serine x2 Linker + Campath-1H heavy chain with wild type human IgG1 constant region. Cloned into expression vector PEE12 (Celltech) for NSO-produced antibody and pRDN-1 and pBAN-2 expression vectors Wellcome for CHO-produced antibody.

### TF CHO/CP-1H IgG1/Link (Linker)

Flexible Glycine4 Serine x2 Linker on CAMPATH-1H light chain V-region + CAMPATH-1H heavy chain with wild type human IgG1 constant region. Cloned into pRDN-1 and pBAN-2 expression vectors (Wellcome) for CHO-produced antibody.

### TF CHO/CP-1H IgG1/MIM-MUTANT 9 (MIM-MUTANT 9-IgG1)

CD52 Mimotope Mutant 9 (QTSAAAVD) tethered to CAMPATH-1H light chain V-region by flexible Glycine4 Serine x2 Linker + CAMPATH-1H heavy chain with wild type human IgG1 constant region. Cloned into expression vectors pRDN-1 and pBAN (Wellcome) for CHO-produced antibody.

### TF CHO/CO-1H IgG1 (CAMPATH-1H)

Wild type CAMPATH-1H light chain V-region + CAMPATH-1H heavy chain with wild type human IgG1 constant region. Cloned into expression vectors pRDN-1 and pBAN-2 (Wellcome) for CHO-produced antibody.

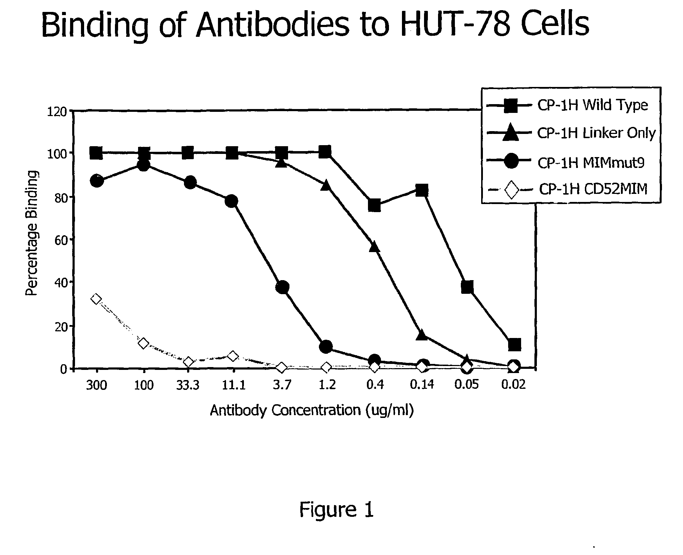

Figure 1 shows the binding abilities of the various antibody constructs to CD52-bearing HUT cells. CP-1H Wild Type has a binding efficiency approximately 5 times greater than binding of CP-1H Linker alone, approximately 100 times greater than CP-MIMmut9, and approximately 10,000 times greater than CP-CD52MIM.

### Effector Function Assessment

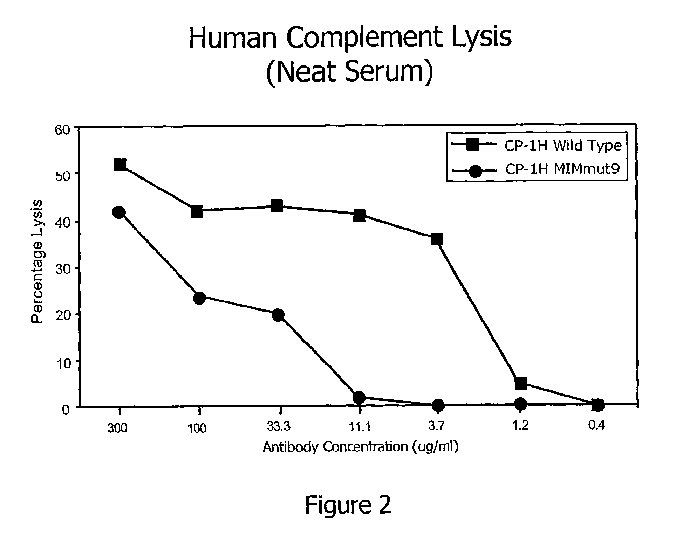

CP-1H MIMmut9 effector function was assessed by testing its ability to kill the CD52 bearing T cell line, HUT78, by complement lysis. CP-1H wild type was used as a positive control. 100 µl of HUT78 cells at 10⁶ cells/ml were mixed with various concentrations of each antibody ranging from 0.4ug/ml to 300ug/ml. This was followed by the addition of 100 µl of fresh, undiluted (neat) human serum as a source of complement. After incubation at 37°C for 45 minutes, propidium iodide was added to the mixture and the cells were analysed by flow cytometry. The percentage of PI stained (lysed) cells was determined for each condition. The results shown in Figure 2 demonstrate that CP-1H MIMmut9 retains the ability to mediate antibody effector function.

The modified and unmodified antibodies were tested to determine induction of cytokines as follows:

### Cytokine Measurement

The purified antibodies were passed over a Detoxi-Gel column (Pierce) to remove endotoxin. All batches of antibody were then tested for endotoxin using the QCL 1000 LAL assay (Bio-Whittaker).

### Ex-Vivo whole blood assay

Blood from healthy laboratory workers was freshly drawn into Heparin. Whole blood samples were incubated with 100, 10 or 1 µg/ml of therapeutic antibody for five hours at 37°c with vigorous shaking. The plasma and cells were then separated by centrifugation.

Plasma TNF-α levels were determined by human TNF-α immunoassay (R&D Systems), plasma IFN-γ levels were determined by human IFN-γ Elisa (BD Bioscience) and plasma IL-6 levels were determined by human IL-6 Elisa (BD Bioscience).
Tables 1 and 2 below report the results of such testing with the modified antibodies reducing release of cytokines.

**Table I**

| TNF-α Levels (pg/ml) in Whole Blood after 5 Hours at 37° C | | | |
|---|---|---|---|
| **Antibody** | **Amount of Antibody** | | |
| | 100 µg/ml | 10 µg/ml | 1 µg/ml |
| CP-LH Wild Type(NSO) | 240 | 245 | >500 |
| CP-1H Wild Type(CHO) | 125 | 250 | >500 |
| CP-1H Linker Only (CHO) | 68 | 145 | 85 |
| CP-1H CD52MIM(NSO) | 0 | 0 | 0 |
| CP-1H CD52MIM(CHO) | 30 | 0 | 0 |
| CP-1H MIMmut9(NSO) | 60 | 28 | 0 |

**Table II**

| Cytokine Levels in Whole Blood Assay after 5 hr incubation at 37° C with antibody | | | |
|---|---|---|---|
| **Antibody (10µg/ml)** | **Cytokin (pg/ml)** | | |
| | TNF-α | IFN-γ | IL-6 |
| CP-1H Wild Type(NSO) | 245 | 262 | NT |
| CP-1H Wild Type (CHO) | 250 | 275 | 31 |
| CP-1H CD52MIM (NSO) | 0 | 0 | 0 |
| CP-1H MIMmut9 (NSO) | 28 | 11 | 10 |

Numerous modifications and variations of the embodiments described herein are possible based on the teachings herein; therefore, the scope of the invention is not limited to such embodiments.

## Claims

1. A pharmaceutical comprising:
(a) a therapeutic antibody that includes an antibody combining site that binds to a therapeutic target, said antibody being modified with a peptide that inhibits binding of the antibody to the therapeutic target, said modified antibody being effective for reducing side effects caused by the antibody and for producing a therapeutic effect by binding to the therapeutic target; and
(b) a pharmaceutical carrier;
wherein the peptide is linked by a linker to the antibody and is reversibly bound to the antibody combining site.

2. A pharmaceutical according to Claim 1, wherein the avidity of the modified antibody combined with the peptide is at least 4-fold less than the avidity of the unmodified antibody and no more than 100-fold less.

3. A pharmaceutical according to Claim 2, wherein the antibody is an aglycosylated antibody.

4. A pharmaceutical according to Claim 3, wherein only one of the chains of the antibody has a peptide linked thereto that binds to the antibody combining site.

5. A pharmaceutical according to Claim 1, wherein the Fc portion of the antibody is aglycosylated.

6. A pharmaceutical according to Claim 1, wherein binding of the antibody to the Fc receptor is essentially eliminated.

7. A pharmaceutical according to Claim 1, wherein the antibody is a non-human antibody.

8. A pharmaceutical according to Claim 1, wherein the antibody is a chimeric antibody.

## Patentansprüche

1. Arzneimittel, umfassend:
(a) einen therapeutischen Antikörper, der eine Antikörperkombinierungsstelle einschließt, die an ein therapeutisches Ziel bindet, wobei der Antikörper mit einem Peptid modifiziert ist, das die Bindung des Antikörpers an das therapeutische Ziel hemmt, wobei der modifizierte Antikörper wirksam zum Verringern von Nebenwirkungen, verursacht durch den Antikörper, und zum Erzeugen einer therapeutischen Wirkung durch Binden an das therapeutische Ziel ist; und
(b) einen pharmazeutischen Träger;
wobei das Peptid durch einen Linker mit dem Antikörper verknüpft ist und reversibel an die Antikörperkombinierungsstelle gebunden ist.

2. Arzneimittel nach Anspruch 1, wobei die Avidität des modifizierten Antikörpers, kombiniert mit dem Peptid, mindestens 4-fach geringer als die Avidität des unmodifizierten Antikörpers und nicht mehr als 100-fach geringer ist.

3. Arzneimittel nach Anspruch 2, wobei der Antikörper ein aglycosylierter Antikörper ist.

4. Arzneimittel nach Anspruch 3, wobei nur eine der Ketten des Antikörpers ein damit verknüpftes Peptid aufweist, das an die Antikörperkombinierungsstelle bindet.

5. Arzneimittel nach Anspruch 1, wobei der Fc-Anteil des Antikörpers aglycosyliert ist.

6. Arzneimittel nach Anspruch 1, wobei die Bindung des Antikörpers an den Fc-Rezeptor im wesentlichen beseitigt ist.

7. Arzneimittel nach Anspruch 1, wobei der Antikörper ein nicht-humaner Antikörper ist.

8. Arzneimittel nach Anspruch 1, wobei der Antikörper ein chimärer Antikörper ist.

## Revendications

1. Produit pharmaceutique comprenant:
(a) un anticorps thérapeutique qui comprend un site de combinaison d'anticorps qui se lie à une cible thérapeutique, ledit anticorps étant modifié avec un peptide qui inhibe la liaison de l'anticorps à la cible thérapeutique, ledit anticorps modifié étant efficace pour réduire les effets secondaires causés par l'anticorps et pour produire un effet thérapeutique en se liant à la cible thérapeutique; et
(b) un excipient pharmaceutique;
dans lequel le peptide est lié à l'anticorps par un lieur et lié de manière réversible au site de combinaison d'anticorps.

2. Produit pharmaceutique selon la revendication 1, dans lequel l'avidité de l'anticorps modifié combiné au peptide est au moins 4 fois moindre que l'avidité de l'anticorps non modifié et pas plus de 100 fois moindre.

3. Produit pharmaceutique selon la revendication 2, dans lequel l'anticorps est un anticorps aglycosylé.

4. Produit pharmaceutique selon la revendication 3, dans lequel seule l'une des chaînes de l'anticorps a un peptide lié à celle-ci qui se lie au site de combinaison d'anticorps.

5. Produit pharmaceutique selon la revendication 1, dans lequel la portion Fc de l'anticorps est aglycosylée.

6. Produit pharmaceutique selon la revendication 1, dans lequel la liaison de l'anticorps au récepteur Fc est essentiellement éliminée.

7. Produit pharmaceutique selon la revendication 1, dans lequel l'anticorps est un anticorps non humain.

8. Produit pharmaceutique selon la revendication 1, dans lequel l'anticorps est un anticorps chimérique.


### SEQUENCE LISTING

<110> Isis Innovation Limited
<120> Therapeutic Antibodies With Reduced Side Effects
<130> WPP88394
<150> US 60/397,934
   <151> 2002-07-23
<160> 8
<170> PatentIn version 3.2
<210> 1
   <211> 24
   <212> DNA
   <213> Artificial Sequence
<220>
   <223> PCR Primer
<400> 1
   cacagatgcg taaggagaaa atac 24
<210> 2
   <211> 19
   <212> DNA
   <213> Artificial Sequence
<220>
   <223> PCR Primer
<400> 2
   gcagtgagcg caacgcaat 19
<210> 3
   <211> 60
   <212> DNA
   <213> Artificial Sequence
<220>
   <223> PCR Primer
<400> 3
   gcttccgcct ccaccggatc cgccacctcc ttgggagtgg acacctgtag ctgttgctac 60
<210> 4
   <211> 56
   <212> DNA
   <213> Artificial Sequence
<220>
   <223> PCR Primer
<400> 4
   ggaggtggcg gatccggtgg aggcggaagc gacatccaga tgacccagag cccaag 56
<210> 5
   <211> 48
   <212> DNA
   <213> Artificial Sequence
<220>
   <223> PCR Primer
<400> 5
   gtctgctgat gggctgctgg tttgggagtg gacacctgta gctgttgc 48
<210> 6
   <211> 48
   <212> DNA
   <213> Artificial Sequence
<220>
   <223> PCR Primer
<400> 6
   caaaccagca gcccatcagc agacggaggt ggcggatccg gtggagga 48
<210> 7
   <211> 48
   <212> DNA
   <213> Artificial Sequence
<220>
   <223> PCR Primer
<400> 7
   gtctactgct gcggcgctgg tttgggagtg gacacctgta gctgttgc 48
<210> 8
   <211> 48
   <212> DNA
   <213> Artificial Sequence
<220>
   <223> PCT Primer
<400> 8
   caaaccagcg ccgcagcagt agacggaggt ggcggatccg gtggagga 48